# EUROPEAN PATENT SPECIFICATION

(11) **EP 0 501 374 B1**
(45) Date of publication and mention of the grant of the patent: **06.09.1995**
(21) Application number: 92103058.1
(22) Date of filing: 24.02.1992
(51) Int. Cl.: C07C 68/08, C07C 69/96

(54) **Process for purifying dimethyl carbonate**
Verfahren zur Reinigung von Dimethylcarbonat
Procédé d'épuration du carbonate de diméthyle

(30) Priority: 26.02.1991 JP 53148/91
(43) Date of publication of application: 02.09.1992
(73) Proprietor: UBE INDUSTRIES, LTD., Ube-shi, Yamaguchi-ken 755 (JP)
(72) Inventor: Nishihira, Keigo, c/o Ube Chemical Factory, Ube-shi, Yamaguchi-ken (JP); Yoshida, Shinichi, c/o Ube Chemical Factory, Ube-shi, Yamaguchi-ken (JP); Tanaka, Shuji, c/o Ube Chemical Factory, Ube-shi, Yamaguchi-ken (JP)
(74) Representative: Hansen, Bernd, Dr. Dipl.-Chem.

(56) References cited:
- EP-A- 0 021 211
- EP-A- 0 139 442
- DE-A- 2 706 684
- Beilsteins Handbuch der Organischen Chemie, 4th Edition (1920), Vol. 2, p. 534 & Drude, Zeitschrift für Physicalische Chemie Ser. A, Vol. 23 (1897), p. 310.
- Beilsteins Handbuch der Organischen Chemie, 4th Edition, 4th Supplement (1976), Vol. 2, part 3, p. 1847.

## Description

This invention relates to a process for purifying dimethyl carbonate which is useful as a synthesis starting material for an aromatic polycarbonate and various chemical products and as a solvent. More particularly, it relates to a process for purifying dimethyl carbonate which comprises distilling off and separating methanol from a mixture of dimethyl carbonate and methanol in the presence of dimethyl oxalate.

As a process for preparing dimethyl carbonate, there have been practiced, for example, the method of reacting carbon monoxide, methanol and an acid by using copper chloride Japanese Patent Publications No. 11129/1970 and No. 45655/1980), the method of interesterifying a cyclic carbonate such as ethylene carbonate with methanol in the presence of a catalyst (U.S. Patents No. 3,642,858 and No. 3,803,201, and Japanese Patent Publication No. 27658/1985), and the method of subjecting carbon monoxide and nitrite to vapor reaction in the presence of a catalyst (Japanese Unexamined Patent Publications No. 274816/1989 and No. 201146/1990). However, in either of the methods, dimethyl carbonate is obtained as a mixture with methanol so that separation from methanol is indispensable for purifying dimethyl carbonate. Dimethyl carbonate and methanol constitute an azeotropic mixture in a composition ratio of 30 : 70 (weight ratio), and thus, it is difficult to separate the mixture by distillation under normal pressure.

Thus, many investigations have been carried out with regard to a method for purifying dimethyl carbonate starting from a mixture of both of the above components, and various proposals have been made. For example, there has been proposed the method of obtaining a crystalline product enriched in dimethyl carbonate by cooling as disclosed in U.S. Patent No. 3,803,201, the method of separating the mixture by distillation and thereby breaking the azeotrope under pressure as disclosed in Japanese Patent Publication No. 3463/1984, and the method of separation by distillation and thereby adding a hydrocarbon such as hexane and heptane as disclosed in Japanese Unexamined Patent Publication No. 41820/1979. However, these methods cannot satisfy the industrial requirements. The method of applying extraction and distillation with water is not economical since dimethyl carbonate dissolves well in water and is easily saponified so that a high loss is caused. An apparatus to be used in the method of distillation under pressure is extremely expensive, and operations therewith are difficult. Also the method of adding hydrocarbon includes complicated operations and is disadvantageous with regard to energy. Finally, the method of precipitation by cooling is industrially not practical.

DE-A-27 06 684 discloses a process for the separation of dimethyl carbonate from its mixtures with methanol by breakage of the azeotropic mixture by an extraction medium having certain properties, i.e. (a) inert, aprotic, organic liquid, (b) having a boiling point above 100°C, (c) fully miscible with dimethyl carbonate, (d) and having a dielectric constant between 4 and 90, and (e) a dipol moment between 1.5 and 5 Debye.

### SUMMARY OF THE INVENTION

An object of the present invention is to provide a process for purifying dimethyl carbonate which can provide dimethyl carbonate with high quality, wherein the azeotropic phenomenon of dimethyl carbonate and methanol is excluded, and methanol can be, distilled and separated easily.

The present inventors have run intensive investigations and have found that dimethyl carbonate and methanol do not constitute an azeotropic composition in the three-component system of dimethyl oxalate, dimethyl carbonate and methanol and thereby accomplished the present invention.

That is, the present invention is a process for purifying dimethyl carbonate which comprises distilling a mixture of dimethyl carbonate and methanol in the presence of dimethyl oxalate to separate and remove methanol from the mixture, wherein dimethyl oxalate is added in an amount of 0.3 or more in terms of the mole fraction.

### BRIEF DESCRIPTION OF THE DRAWINGS

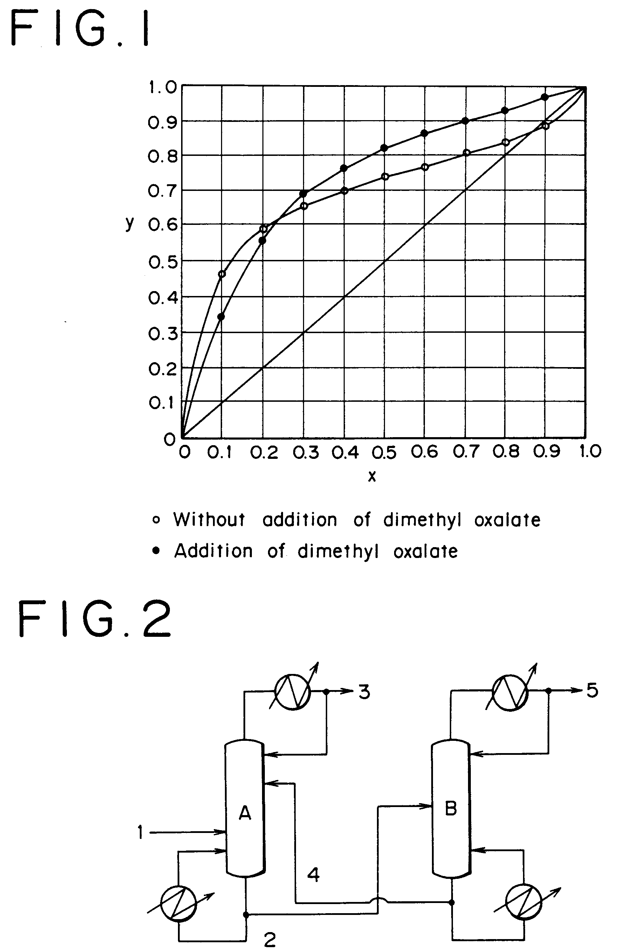

Fig. 1 is a graph showing the measured results of gas-liquid equilibrium of dimethyl carbonate and methanol, and Fig. 2 shows a processing diagram of the continuous preparation process.

### DESCRIPTION OF THE PREFERRED EMBODIMENTS

In the following, the present invention is described in more detail.

The amount of dimethyl carbonate and methanol is not particularly limited. The amount of dimethyl oxalate to be added is 0.3 or more, preferably 0.5 or more, in terms of the mole fraction of dimethyl oxalate in the three components-systems in order to enhance separation efficiency. With regard to the separation efficiency, there is no upper limit thereof, and a range which can be industrially practiced, is represented e.g. by 0.8 to 0.9 in terms of the mole fraction of dimethyl oxalate in the three components-system.

In the three component system of dimethyl carbonate, dimethyl oxalate and methanol, dimethyl carbonate and methanol do not show the azeotropic phenomenon, so that methanol can be separated by distillation under normal pressure. Next, the remaining mixture of dimethyl carbonate and dimethyl oxalate can be separated by normal distillation to give separated dimethyl carbonate. The remaining dimethyl oxalate can be used again by circulation.

An industrial process according to the present invention can be practiced by using a continuous apparatus as shown in Fig. 2. In Fig. 2, a crude reaction mixture (1) which is a mixture of dimethyl carbonate and methanol is continuously fed to a first column can (A) and also a solution of dimethyl oxalate is fed therein continuously. The mixture is refluxed by heating the bottom of the column to effect continuous distillation and methanol is taken out from the top of the first column as distilled solution (3). The solution (2) at the bottom of the first column can is a mixture of dimethyl carbonate and dimethyl oxalate and is fed to a second column can (B) and refluxed to effect continuous distillation whereby dimethyl carbonate is taken out from the top of the second column as distilled solution (5.) The solution (4) at the bottom of the second tower can is dimethyl oxalate as a distillation residue and is returned to the first column can (A) for circulated use whereby a continuous operation can be effected.

When the process of the present invention is effected in the presence of water existing in the distillation system, dimethyl oxalate is hydrolyzed so that water should previously be removed, if necessary. In the processes for preparing dimethyl carbonate as mentioned above, water is substantially not contained in the reaction product obtained by the method using a cyclic carbonate or the method using a nitrite. Also, in the latter method, dimethyl oxalate is contained as a by-product in a little amount so that the reaction product prepared by this method can be applied to the purifying process of the present invention directly. For the above reason, dimethyl carbonate can be prepared advantageously by the latter method as compared to the reaction product obtained by the other method.

In the three-component system comprising dimethyl oxalate, dimethyl carbonate and methanol according to the process of the present invention, dimethyl carbonate and methanol do not constitute azeotropic material. Fig. 1 is a graph of the measured results of gas-liquid equilibrium showing the matter, and it shows that by addition of dimethyl oxalate, separation by distillation of dimethyl carbonate and methanol becomes easy.

### EXAMPLES

In the following, the process of the present invention will be explained specifically by referring to Examples, but the present invention is not limited by these Examples.

### Example 1

To the 6th step from the bottom of a 50 steps oldershaw having an inner diameter of 32 mm was continuously fed a solution of 70 % by weight of methanol and 30 % by weight of dimethyl carbonate in an amount of 120 g per hour. At the same time, to the 6th step from the top thereof dimethyl oxalate dissolved by heating was continuously fed in an amount of 950 g per hour. Continuous operation was carried out by heating the bottom and operating with a reflux ratio of about 5, and when the system has turned to a steady state (top temperature: 64.7°C, bottom temperature: 151.3 °C), the solution distilled from the top and the bottom solution were analyzed by gas chromatography. As the results, the distilled solution from the top contained 98.8 % by weight of methanol and 1.2 % by weight of dimethyl carbonate and the bottom solution contained 0.1 % by weight of methanol, 3.5 % by weight of dimethyl carbonate and 96.4 % by weight of dimethyl oxalate. Also, the distilled amount at the top and the amount taken out from the bottom at the steady state were 83 g/hr and 986 g/hr, respectively.

### Example 2

The bottom solution taken out in Example 1 was continuously fed to the 25th step from the top of the same shape oldershaw in an amount of 986 g per hour and continuous distillation was carried out with a reflux ratio of 4.7. When the system has turned to a steady state (top temperature: 86.2°C, bottom temperature: 166.4 °C), the distilled solution from the top and the bottom solution taken out were analyzed by gas chromatography. As the results, the distilled solution from the top contained 2.8 % by weight of methanol and 97.2 % by weight of dimethyl carbonate and the distilled amount was 35 g/hr. Also, the bottom solution contained substantially 100 % by weight of dimethyl oxalate and the amount which was taken out was 949 g/hr.

### Example 3

The same procedure was carried out as in Example 1 except for changing the composition of the feed solution to 21.3 % by weight of methanol and 78.7 % by weight of dimethyl carbonate, and the amount of dimethyl oxalate to be fed was 400 g per hour. As the results, the flow amount of the distilled solution at the top was 26 g/hr and the composition thereof was 97.7 % by weight of methanol and 2.3 % by weight of dimethyl carbonate. Also, the amount of the bottom solution, which was taken out, was 493 g/hr and the composition thereof was 0.04 % by weight of methanol, 19.0 % by weight of dimethyl carbonate and 81.0 % by weight of dimethyl oxalate.

### Comparative example 1 (the case that dimethyl oxalate is not added)

A solution of 70 % by weight of methanol and 30 % by weight of dimethyl carbonate was continuously fed without feed of dimethyl oxalate as in Example 1, and then the bottom was heated. The continuous operation was carried out at a reflux ratio of about 5, and when the system has turned to a steady state, the top distilled solution and the bottom solution were analyzed by a gas chromatography. As a result, both top distilled solution and bottom solution had a composition of 70 % by weight of methanol and 30 % by weight of dimethyl carbonate.

According to the present invention methanol can be distilled and separated under normal pressure by breaking the azeotrope of dimethyl carbonate and methanol, and the added dimethyl oxalate can be recovered and circulated, and the process can be carried out continuously. Therefore, the process of the present invention can be used in an excellent way as an industrial process.

## Claims

1. A process for purifying dimethyl carbonate which comprises distilling a mixture of dimethyl carbonate and methanol in the presence of dimethyl oxalate to separate and remove methanol, wherein dimethyl oxalate is added in an amount of 0.3 or more in terms of the mole fraction.

2. The process according to claim 1, wherein dimethyl oxalate is added in an amount of 0.5 or more in terms of the mole fraction.

3. The process according to claim 1, wherein the distillation is carried out under normal pressure.

4. The process according to claim 1, wherein said dimethyl carbonate results from a preparation method using a nitrite.

5. The process according to claim 1, wherein the process comprises continuously feeding a crude reaction mixture (1) which is a mixture of dimethyl carbonate and methanol to a first column can (A) and simultaneously feeding a solution of dimethyl oxalate continuously, refluxing the mixture by heating the bottom of the column to effect continuous distillation, taking out methanol from the top of the first column as distilled solution (3), feeding from the bottom of a first column can (A) the solution (2) which is a mixture of dimethyl carbonate and dimethyl oxalate to a second column can (B), refluxing the mixture to effect continuous distillation, taking out dimethyl carbonate from the top of the second column as distilled solution (5), and returning from the bottom of the second column a second tower can solution (4) which is dimethyl oxalate as a distillation residue to the first column can (A) for circulated use and thereby effecting continuous operation.

## Patentansprüche

1. Verfahren zur Reinigung von Dimethylcarbonat, das das Destillieren einer Mischung aus Dimethylcarbonat und Methanol in Gegenwart von Dimethyloxalat zum Abtrennen und Entfernen des Methanols umfaßt, worin Dimethyloxalat in einer Menge von 0,3 oder mehr, ausgedrückt als Molenbruch, zugegeben wird.

2. Verfahren gemäß Anspruch 1, worin Dimethyloxalat in einer Menge von 0,5 oder mehr, ausgedrückt als Molenbruch, zugegeben wird.

3. Verfahren gemäß Anspruch 1, worin die Destillation unter Normaldruck ausgeführt wird.

4. Verfahren gemäß Anspruch 1, worin das Dimethylcarbonat aus einem Herstellungsverfahren, das ein Nitrit verwendet, resultiert.

5. Verfahren gemäß Anspruch 1, worin das Verfahren umfaßt: das kontinuierliche Einspeisen einer rohen Reaktionsmischung (1), die eine Mischung aus Dimethylcarbonat und Methanol ist, zu einer ersten Kolonne (A) und gleichzeitig das kontinuierliche Einspeisen einer Dimethyloxalat-Lösung, das Rückflussieren der Mischung unter Erhitzen der Kolonnenblase, um eine kontinuierliche Destillation zu bewirken, das Abziehen von Methanol am Kopf der ersten Kolonne als destillierte Lösung (3), das Einspeisen der Lösung (2), die eine Mischung aus Dimethylcarbonat und Dimethyloxalat ist, von der Blase einer ersten Kolonne (A) zu einer zweiten Kolonne (B), das Rückflussieren der Mischung, um eine kontinuierliche Destillation zu bewirken, das Abziehen des Dimethylcarbonats am Kopf der zweiten Kolonne als destillierte Lösung (5), und das Rückführen der Lösung (4) des zweiten Kolonnenturms, die ein Dimethyloxalat-Destillationsrückstand ist, von der Blase der zweiten Kolonne zu der ersten Kolonne (A) unter Kreisführung und dadurch das Bewirken einer kontinuierlichen Betriebsweise.

## Revendications

1. Procédé de purification du carbonate de diméthyle qui comprend la distillation d'un mélange de carbonate de diméthyle et de méthanol en présence d'oxalate de diméthyle en vue de séparer et d'éliminer le méthanol, procédé dans lequel la fraction molaire de l'oxalate de diméthyle ajouté est égale à 0,3 ou plus.

2. Procédé selon la revendication 1, dans lequel la fraction molaire de l'oxalate de diméthyle ajouté est égale à 0,5 ou plus.

3. Procédé selon la revendication 1, dans lequel la distillation est conduite sous pression ordinaire.

4. Procédé selon la revendication 1, dans lequel le carbonate de diméthyle est obtenu par un procédé de préparation employant un nitrite.

5. Procédé selon la revendication 1, dans lequel le procédé comprend le chargement en continu d'un mélange réactionnel brut (1) qui est un mélange de carbonate de diméthyle et de méthanol dans une première cuve en forme de colonne (A ) et le chargement simultané en continu d'une solution de diméthyle d'oxalate, le chauffage à reflux du fond de la colonne pour réaliser une distillation en continu, le prélèvement du méthanol à partir du sommet de la première colonne à l'état de solution distillée (3), le chargement à partir du fond de la cuve en forme de colonne (A) de la solution (2) qui est un mélange de carbonate de diméthyle et d'oxalate de diméthyle dans une seconde cuve en forme de colonne (B), le chauffage à reflux du mélange pour réaliser une distillation en continu, le prélèvement du carbonate de diméthyle à partir du sommet de la seconde colonne à l'état de solution distillée (5), et le retour dans la première cuve en forme de colonne (A) de la solution contenue dans le fond de la seconde cuve (4) qui est un résidu de distillation formé d'oxalate de diméthyle à des fins de recyclage de manière à permettre l'exécution des opérations en continu.
